# EUROPÄISCHE PATENTSCHRIFT

(11) **EP 0 873 088 B1**
(45) Veröffentlichungstag und Bekanntmachung des Hinweises auf die Patenterteilung: **07.03.2001**
(21) Anmeldenummer: 96946324.9
(22) Anmeldetag: 23.12.1996
(51) Int. Cl.: A61B 18/12

(54) **HF-ELEKTRODE FÜR EIN MONOPOLAR ARBEITENDES HF-INSTRUMENT**
HIGH FREQUENCY (HF) ELECTRODE FOR A HF INSTRUMENT OPERATING IN MONOPOLAR MODE
ELECTRODE HF POUR INSTRUMENT HF DE MODE MONOPOLAIRE

(30) Priorität: 22.12.1995 DE 19548493
(43) Veröffentlichungstag der Anmeldung: 28.10.1998
(73) Patentinhaber: Karl Storz GmbH & Co. KG, 78532 Tuttlingen (DE)
(72) Erfinder: GMINDER, Frank, D-78647 Trossingen (DE)
(74) Vertreter: Münich, Wilhelm, Dr.
(86) Internationale Anmeldenummer: DE9602471
(87) Internationale Veröffentlichungsnummer: WO9723168

(56) Entgegenhaltungen:
- DE-A- 2 521 719
- DE-U- 29 519 844
- FR-A- 825 301

## Beschreibung

### Technisches Gebiet

Die Erfindung bezieht sich auf eine HF-Elektrode für ein monopolar arbeitendes HF-Instrument gemäß dem Oberbegriff des Patentanspruchs 1.

Derartige Elektroden werden beispielsweise in HF-Resektoskopen zur Behandlung des Prostata-Adinom-Gewebes eingesetzt.

### Stand der Technik

Mit den herkömmlichen schlingenförmigen Elektroden, die aus einem dünnen Draht mit einem Durchmesser von typischerweise bis zu 1 mm oder entsprechendem Flachmaterial bestehen, tritt abhängig von der verwendeten Stromart - Schneidemodus, Koagulationsmodus, Spraykoagulationsmodus - des HF-Generators ein Schneide-und Oberflächenkoagulationseffekt auf, der zur Blutstillung angeschnittener Blutgefäße verwendet wird. Ein in der Praxis nutzbarer Vaporisations-Effekt tritt praktisch nicht auf.

Weiterhin hat die Modulation des Stromes Einfluß auf das "Bearbeitungsergebnis": Abhängig von der Modulation des "Schneidestromes" kann zusätzlich zum Schneideeffekt eine Oberflächenkoagulation erzeugt werden, die eine Blutstillung oberflächlich verlaufender Blutgefäße ermöglicht.

Großflächige Kugel- oder Walzenelektroden, an die "Koagulationsstrom" angelegt ist, dienen in der Regel lediglich zur großflächigen, operationsabschließenden Blutstillung.

Als eine Alternative zur HF-Strom-Gewebeabtragung gibt es die Möglichkeit der Gewebeablation mittels Laser. Hierfür geeignete Laser sind aber wesentlich teurer als HF-Generatoren, so daß nach Wegen gesucht worden ist, auch mit HF-Elektroden möglichst blutarm das Adinom-Gewebe abtragen zu können.

Eine Reihe von Autoren hat vorgeschlagen, herkömmliche monopolare Elektroden mit einer zylinderförmigen Rolle zu verwenden. Nur exemplarisch wird hierzu auf die US-PS 5 395 363 verwiesen, von der bei der Formulierung des Oberbegriffs des Patentanspruchs 1 ausgegangen wird.

Die Oberfläche der zylinderförmigen Rolle kann dabei auf die verschiedensten Arten ausgebildet sein: So sind Rollen mit glatter Oberfläche, mit gerillter Oberfläche oder mit Spitzen bekannt.

Die Verwendung von HF-Elektroden mit Rollen hat jedoch den Nachteil, daß zum einen die vergleichsweise große Rolle die Sicht Operateurs einschränkt. Zum anderen ist es mit derartigen Rollen in der Praxis nur möglich, das Gewebe durch Zufuhr hoher HF-Leistungen zu vaporisieren, wobei die Effizienz unbefriedigend ist. Dies bedeutet, daß der Patient nicht nur eine erhebliche Zeit einem sehr hohen und damit mit einem großen Gefahren-potential behafteten Stromfluß bzw. sehr hohen Energien ausgesetzt ist, sondern daß auch die Narkosezeit deutlich gegenüber anderen Operationstechniken verlängert wird.

### Darstellung der Erfindung

Der Erfindung liegt die Aufgabe zugrunde, eine HF-Elektrode für ein monopolar arbeitendes HF-Instrument anzugeben, mit der die Operationszeit dadurch verkürzt wird, daß das Gewebe sowohl vaporisiert als auch geschnitten werden kann.

Eine erfindungsgemäße Lösung dieser Aufgabe ist im Patentanspruch 1 angegeben. Weiterbildungen der Erfindung sind Gegenstand der Ansprüche 2ff.

Erfindungsgemäß ist erkannt worden, daß es mit einer Elektrode, die prinzipiell die Kontur einer bekannten Schlinge hat, dann möglich ist, sowohl zu vaporisieren als auch zu schneiden, wenn diese Elektrode einen zentralen Teil aufweist, der eine Längserstreckung, d.h. eine Erstreckung in Richtung der Instrumentenachse bzw. in Richtung der Längsachse der Zuleitung bzw. der Zuleitungen von wenigstens 2 mm und nicht mehr als 6 mm aufweist. Die Bügel, die den zentralen Teil mit der Zuleitung bzw. den Zuleitungen verbinden, bestehen in an sich bekannter Weise aus einem Drahtmaterial mit wesentlich geringerer Längserstreckung, die gemäß Anspruch 3 nicht mehr als 1,5 mm, bevorzugt nicht mehr als 1 mm beträgt.

Herkömmliche Schlingen bestehen dagegen durchgehend aus Materialien mit den gleichen Abmessungen und insbesondere aus Drahtmaterialien, wobei die Längserstreckung bzw. der Durchmesser des Drahtes typischerweise 1 mm beträgt.

Mit der erfindungsgemäßen Elektrode kann gleichzeitig "blutarm" geschnitten und das Gewebe vaporisiert werden. Aufgrund der erfindungsgemäßen Ausgestaltung des zentralen Teils der Schlinge fließt großflächig - aber dennoch limitiert - HF-Strom in das Gewebe und bewirkt während des Schneidevorgangs einen Koagulations- und Vaporisationsprozeß ! Dennoch ist es möglich, mit der erfindungsgemäßen Elektrode zu "graben" bzw. zu "baggern", um Gewebe abzuschneiden.

Die gezielte Anwendung der seitlichen Bügel mit vergleichsweise geringer Längserstreckung erlaubt die Verwendung der erfindungsgemäß ausgebildeten HF-Elektrode mit den herkömmlichen dünnen Schlingenelektroden eigenen Vorteilen, nämlich dem Ablösen bzw. Abschneiden "gestielter" Gewebeteile.

Dabei ist es bevorzugt, wenn die Bügel nicht aus einem Drahtmaterial mit rundem Querschnitt, sondern gemäß Anspruch 2 aus einem bandförmigen Material bzw. einem Material mit einem elliptischen Querschnitt bestehen, dessen längere Achse sich in Richtung der Längsachse der Zuleitung erstreckt.

Bei der im Anspruch 4 angegebenen Weiterbildung, bei der die Zuleitung, die Bügel und der zentrale Teil in Art eines "Z" angeordnet sind, erleichtert die Reinigung der erfindungsgemäßen Elektrode von abgeschnittenen Gewebeteilen.

Die im Anspruch 9 beanspruchte Weiterbildung der erfindungsgemäßen HF-Elektrode mit einer geschärften Schneidekante ermöglicht einen beim Anschneiden im Gewebe optimierten Stromfluß von der Schneidekante in das Gewebe. Die dabei an der Kante auftretende hohe Stromdichte bewirkt ein optimales Anschneiden des Gewebes. Nach dem Anschneiden wird die Elektrode so gehalten, daß der Stromfluß im wesentlichen senkrecht von der "Platten-Oberfläche" in das Gewebe erfolgt.

Die Möglichkeit, mit der "hinteren" Kante des zentralen Teils zu schneiden, wird dadurch unterstützt, daß der zentrale Teil einen angenähert elliptischen Längsschnitt aufweist (Anspruch 10).

Da die erfindungsgemäße Elektrode im wesentlichen die Grundform einer Schlinge hat, wird durch sie die Sicht des Operateurs auf das Operationsgebiet nicht eingeschränkt.

Bei einer weiteren bevorzugten und im Anspruch 5 angegebenen Ausführungsform ist der zentrale Teil in Querrichtung geformt und insbesondere gebogen. Hierdurch wird eine großflächige Auflage auf dem zu behandelnden Gewebe unterstützt und gleichzeitig die Sicht beispielsweise durch ein in ein Resktoskop eingesetztes Endoskop nicht eingeschränkt.

Durch die bevorzugte Breite des zentralen Teils von wenigstens (ca.) 3,5 mm und bevorzugt von wenigstens 4,5 mm ergibt sich eine besonders wirkungsvolle Vaporisation des Gewebes während des Schneidens (Anspruch 6).

Dabei ist es gemäß Anspruch 8 von Vorteil, wenn der zentrale Teil Rillen aufweist, da durch die Rillen der bei der Vaporisation entstehende "Dampf" entweichen kann, und die Möglichkeit des Stromübertrittes in das Gewebe optimiert wird.

Die erfindungsgemäße HF-Elektrode kann in an sich bekannter Weise hergestellt werden und insbesondere aus Titan bestehen (Anspruch 11).

Bei einer weiteren bevorzugten Ausführungsform ist der zentrale Teil an seiner Oberseite isoliert, so daß ein Stromfluß nur durch die untere Seite des zentralen - plattenförmig ausgebildeten - Teils auftritt. Hierdurch wird der zur Vaporisation und Koagulation dienende Stromfluß auf den notwendigen Bereich beschränkt und die Strombelastung des Patienten gesenkt (Anspruch 7).

### Kurze Beschreibung der Zeichnung

Die Erfindung wird nachstehend anhand eines Ausführungsbeispieles unter Bezugnahme auf die Zeichnung näher beschrieben, in der zeigen
- Fig. 1: perspektivisch schematisch eine erfindungsgemäße HF-Elektrode, und
- Fig. 2: a bis c ein praktisches Ausführungsbeispiel einer erfindungsgemäßen HF-Elektrode.

### Darstellung von Ausführungsbeispielen

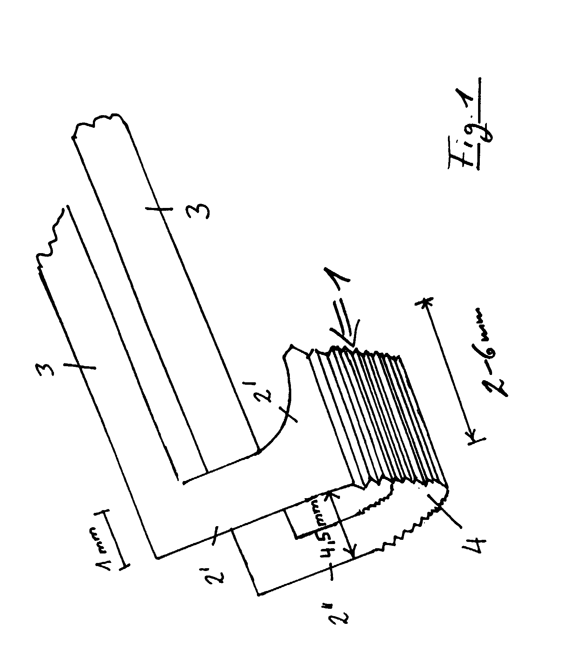

In Fig. 1 ist eine erfindungsgemäße HF-Elektrode schematisch dargestellt. Die Elektrode weist einen zentralen Teil 1 und zwei Bügel 2' bzw. 2" auf, die den zentralen Teil mit einer isolierten Zuleitung 3, die bei dem gezeigten Ausführungsbeispiel aus zwei Stangen besteht, verbindet. Über die isolierte Zuleitung 3 ist die HF-Elektrode beispielsweise mit einem Standard-Resektoskop (nicht dargestellt) verbunden, das wiederum mit einem ebenfalls nicht dargestellten HF-Generator verbunden ist. Dieser HF-Generator ist bevorzugt ein geregelter Generator. Als Resektoskope und HF-Generatoren können Instrumente bzw. Geräte eingesetzt werden, wie sie von der Anmelderin, der Karl Storz GmbH & Co, Tuttlingen, Deutschland serienmäßig hergestellt und vertrieben werden.

Der zentrale Teil 1 der erfindungsgemäßen HF-Elektrode hat eine Längserstreckung von 2 mm bis 6 mm, bevorzugt von 3 bis 5 mm. Die Breite des zentralen Teils 1 beträgt typischerweise ungefähr 5 mm.

Die Bügel bestehen aus einem Drahtmaterial oder einem bandförmigen Material, das einen Durchmesser bzw. eine Erstreckung in Richtung der Längsachse der Zuleitung 3 von typischerweise 1 mm aufweist. Damit ist es möglich, in bestimmten Fällen auch mit einem der Bügel einen Schnitt durch das Gewebe auszuführen.

Weiterhin weist der zentrale Teil an seiner Oberseite eine Isolation 4 und an seiner Unterseite Rillen auf.

Durch die Isolation 4 wird der Stromfluß dadurch begrenzt, daß Strom nur von der Unterseite, nicht jedoch von der Oberseite des zentralen Teils in das Gewebe eintritt.

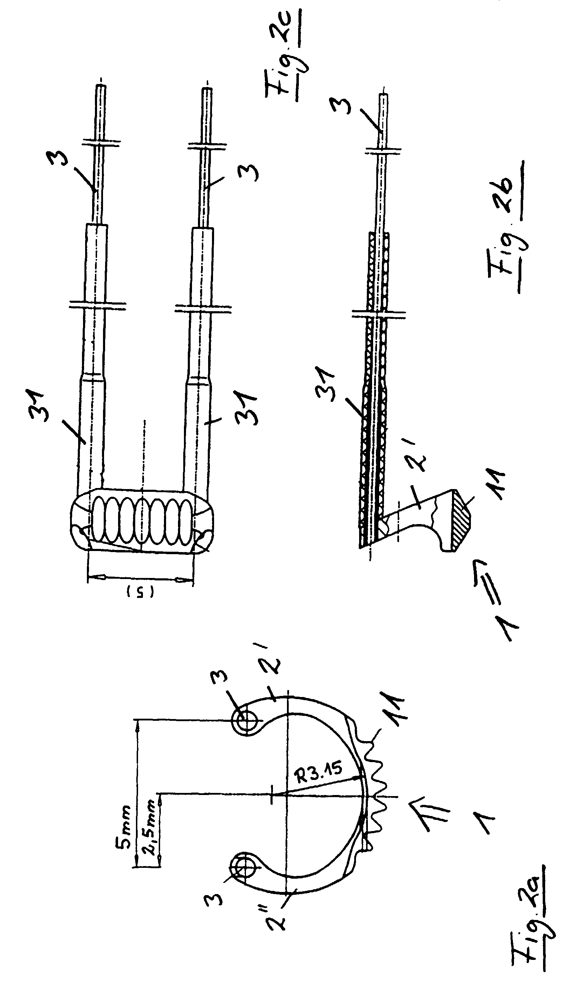

Die Fig. 2a bis 2c zeigen in einer Vorderansicht (Fig. 2a), einer Schnittansicht (von der Seite, Fig. 2b) und einer Ansicht von unten (Fig. 2c) ein reales Ausführungsbeispiel einer erfindungsgemäßen Elektrode. Gleiche bzw. entsprechende Teile wie in Fig. 1 sind mit den selben Bezugszeichen versehen. Zusätzlich sind mit den Bezugszeichen 31 eine Isolation der Zuleitungen und mit 11 die Rillen an der Unterseite des plattenförmigen Teils 1 bezeichnet.

In den Fig. 2a und 2b sind Maße eingetragen, die besonders bevorzugt sind. Die Fig. 2b und 2c sind im selben Maßstab ausgeführt, so daß die restlichen Maße aus den Figuren entnommen werden können.

Mit der erfindungsgemäßen HF-Elektrode kann sowohl die Vaporisationstechnik als auch die herkömmliche Schneidtechnik angewandt werden. Damit entfallen sämtliche Probleme, die durch einen Elektrodenwechsel auftreten können, wie er beim Stand der Technik erforderlich ist.

Weiterhin kann der sehr hohe und damit mit einem Gefahren-potential behaftete Stromfluß gezielt nur dann eingesetzt werden, wenn er tatsächlich benötigt wird.

Durch die gleichzeitge Anwendung von Schneide- und Koagulations- bzw. Vaporationstechnik ist die erfindungsgemäße HF-Elektrode sehr effizient, so daß die die Narkosezeiten gegenüber anderen Operationstechniken deutlich verkürzt werden.

## Patentansprüche

1. HF-Elektrode mit
- einer isolierten Zuleitung (3), die sich in Richtung der Längsachse eines monopolar arbeitenden HF-Instrument erstreckt, und
- einem Elektrodenteil, der elektrisch leitend mit der Zuleitung verbunden ist und mit dem zu bearbeitenden Gewebe in Eingriff gebracht wird und der aus zwei Bügeln (2', 2'') und einem zentralen Teil (1) besteht,
dadurch **gekennzeichnet**, daß der zentrale Teil (1) in Art einer Platte ausgebildet ist, die eine Längserstreckung, d.h. eine Erstreckung in Richtung der Längsachse des Instruments bzw. der Zuleitung (3) von 2 bis 6 mm hat, und daß die Bügel (2',2") aus einem Drahtmaterial mit wesentlich geringerer Längserstreckung bestehen.

2. Elektrode nach Anspruch 1,
dadurch **gekennzeichnet**, daß die Bügel aus einem bandförmigen Material bzw. einem Material mit einem elliptischen Querschnitt bestehen, dessen längere Achse sich in Richtung der Längsachse der Zuleitung erstreckt.

3. Elektrode nach Anspruch 1 oder 2,
dadurch **gekennzecihnet**, daß die Bügel (2', 2") eine Längserstreckung von nicht mehr als 1,5 mm, bevorzugt 1 mm aufweisen.

4. Elektrode nach einem der Ansprüche 1 bis 3,
dadurch **gekennzeichnet**, daß die Zuleitung (3), die Bügel (2', 2") und der zentrale Teil (1) in Art eines "Z" angeordnet sind.

5. Elektrode nach einem der Ansprüche 1 bis 4,
dadurch **gekennzeichnet**, daß der zentrale Teil in Querrichtung geformt und inbesondere gebogen ist.

6. Elektrode nach einem der Ansprüche 1 bis 5,
dadurch **gekennzeichnet**, daß der zentrale Teil eine Breite von wenigstens 3,5 mm und bevorzugt von wenigstens 4,5 mm hat.

7. Elektrode nach einem der Ansprüche 1 bis 6,
dadurch **gekennzeichnet**, daß der zentrale Teil (1) an seiner Oberseite eine Isolation (4) aufweist.

8. Elektrode nach einem der Ansprüche 1 bis 7,
dadurch **gekennzeichnet**, daß der zentrale Teil an seiner Unterseite Rillen (11) aufweist.

9. Elektrode nach einem der Ansprüche 1 bis 8,
dadurch **gekennzeichnet**, daß die dem proximalen Bereich des HF-Instruments zugewandte Kante des zentralen Teil derart geformt ist, daß sie ein Schneiden erlaubt.

10. Elektrode nach einem der Ansprüche 1 bis 9,
dadurch **gekennzeichnet**, daß der zentrale Teil einen angenähert elliptischen Längsschnitt hat.

11. Elektrode nach einem der Ansprüche 1 bis 10,
dadurch **gekennzeichnet**, daß sie aus Titan besteht.

12. Elektrode nach einem der Ansprüche 1 bis 11,
dadurch **gekennzeichnet**, daß der zentrale Teil (1) eine Längserstreckung von 3 bis 5 mm hat

## Claims

1. HF electrode comprising
- an insulated feeder line (3) extending along the longitudinal axis of an HF instrument operating in a monopolar mode, and
- an electrode part which is connected to said feeder line in an electrically conducting manner and which is engaged with the tissue to be processed, and which consists of two straps (2', 2") and a central portion (1),
**characterised** in that said central portion (1) is configured in the manner of a plate having a longitudinal extension, i.e. an extension along the longitudinal axis of the instrument or said feeder line (3), respectively, of 2 to 6 mm, and that said straps (2', 2") consist of a wire material having a substantially shorter longitudinal extension.

2. Electrode according to Claim 1,
**characterised** in that said straps consist of a strip-shaped material or a material having an elliptic cross-section, whose longer axis extends along the longitudinal axis of said feeder line.

3. Electrode according to Claim 1 or 2,
**characterised** in that said straps (2', 2") have a longitudinal extension not exceeding 1.5 mm, preferably 1 mm.

4. Electrode according to any of the Claims 1 to 3,
**characterised** in that said feeder line (3), said straps (2', 2") and said central portion (1) are disposed in the manner of a "Z".

5. Electrode according to any of the Claims 1 to 4,
**characterised** in that said central portion is shaped in the transverse direction and is bent in particular.

6. Electrode according to any of the Claims 1 to 5,
**characterised** in that said central portion has a width of at least 3,5 mm and preferably of at least 4.5 mm.

7. Electrode according to any of the Claims 1 to 6,
**characterised** in that said central portion (1) comprises an insulation (4) on its upper side.

8. Electrode according to any of the Claims 1 to 7,
**characterised** in that said central portion presents grooves (11) on its underside.

9. Electrode according to any of the Claims 1 to 8,
**characterised** in that the edge of said central portion, which faces the proximal region of the HF instrument, is shaped so as to permit cutting.

10. Electrode according to any of the Claims 1 to 9,
**characterised** in that said central portion has an approximately elliptic longitudinal section.

11. Electrode according to any of the Claims 1 to 10,
**characterised** in that it is made of titanium.

12. Electrode according to any of the Claims 1 to 11,
**characterised** in that said central portion (1) presents a longitudinal extension of 3 to 5 mm.

## Revendications

1. Électrode H.F. comprenant
- une conduite d'amenée isolée (3), qui s'étend le long de l'axe longitudinal d'un instrument H.F., qui fonctionne en un mode monopolaire, et
- un élément d'électrode, qui est relié à ladite conduite d'amenée de façon électriquement conductrice, et qui se trouve en prise dans le tissu à traiter, et qui est formé par deux brides (2', 2") et une partie centrale (1),
**caractérisé** en ce que ladite partie centrale (1) est configurée de la manière d'une plaque à une étendue longitudinale, c'est-à-dire une étendue le long de l'axe de l'instrument ou respectivement de ladite conduite d'amenée (3), de 2 à 6 mm, et
en ce que lesdites brides (2', 2") consistent en un matériau en fil à une étendue longitudinale essentiellement plus courte.

2. Électrode selon la revendication 1,
**caractérisé** en ce que lesdites brides consistent en un matériau sous forme de bande ou un matériau à une coupe transversale elliptique, dont le plus long axe s'étend le long de l'axe longitudinal de ladite conduite d'amenée.

3. Électrode selon la revendication 1 ou 2,
**caractérisé** en ce que lesdites brides (2', 2") ont une étendue longitudinale inférieure à 1,5 mm, de préférence 1 mm.

4. Électrode selon une quelconque des revendications 1 à 3,
**caractérisé** en ce que ladite conduite d'amenée (3), lesdites brides (2', 2") et ladite partie centrale (1) sont disposées sous la forme d'un "Z".

5. Électrode selon une quelconque des revendications 1 à 4,
**caractérisé** en ce que ladite partie centrale est formée en sens transversal et courbée en particulier.

6. Électrode selon une quelconque des revendications 1 à 5,
**caractérisé** en ce que ladite partie centrale a une largeur d'au moins 3,5 mm et de 4,5 mm de préférence.

7. Électrode selon une quelconque des revendications 1 à 6,
**caractérisé** en ce que ladite partie centrale (1) comprend une isolation (4) à sa face supérieure.

8. Électrode selon une quelconque des revendications 1 à 7,
**caractérisé** en ce que ladite partie centrale présente des rainures (11) de son côté inférieur.

9. Électrode selon une quelconque des revendications 1 à 8,
**caractérisé** en ce que l'arête de ladite partie centrale, que se trouve en face de la région proximale de l'instrument H.F., est formée de façon à permettre le coupage.

10. Électrode selon une quelconque des revendications 1 à 9,
**caractérisé** en ce que ladite partie centrale a une coupe longitudinale approximativement elliptique.

11. Électrode selon une quelconque des revendications 1 à 10,
**caractérisé** en ce quelle est faite en titane.

12. Électrode selon une quelconque des revendications 1 à 11,
**caractérisé** en ce que ladite partie centrale (1) présente une étendue longitudinale de 3 à 5 mm.
